# EUROPEAN PATENT APPLICATION

(11) **EP 4 650 752 A1**
(43) Date of publication of application: **19.11.2025**
(21) Application number: 24175509.9
(22) Date of filing: 13.05.2024
(51) Int. Cl.: G01N 21/25, G01N 33/49, G01N 21/31

(54) **METHOD FOR DETERMINING A SAMPLE QUALITY OF A BIOLOGICAL SAMPLE INSIDE A CONTAINER**

(71) Applicant: Beckman Coulter, Inc., Brea, CA 92821 (US)
(72) Inventor: Windisch, Stephan, 85622 Feldkirchen (DE)
(74) Representative: Maiwald GmbH

(57) **Abstract**

The disclosure relates to a method (100) for determining a sample quality of a biological sample (2) inside an uncapped container (1), the method (100) comprising:
- determining, by a data processing system (11), at least one sample color of the biological sample (2) based on a color image (9) showing at least part of a surface (6) of the biological sample (2) from a top view perspective on the container (1); and
- determining, by the data processing system (11), the sample quality of the biological sample (2) based on a comparison of the determined at least one sample color with at least one reference color.

## Description

### TECHNICAL FIELD

The present invention generally relates to the field of determining sample quality of biological samples, such as body fluid samples, e.g., blood samples, inside containers, such as tubes. Specifically, the present invention relates to a method for determining sample quality of a biological sample inside a container, a computer program product, a data processing system, and a system for determining a sample quality of a biological sample inside a container.

### BACKGROUND

Various types of tests related to patient diagnosis and therapy can be performed by analysis of patient samples or, in other words, biological samples by lab or laboratory automation systems (LAS), which may handle pre-analytical and/or post-analytical stage processing of the samples. Also, the LAS may be connected to one or more analyzers for analytical stage processing of the samples. Any type of biological sample, in particular body fluid sample, may be handled, e.g., urine, blood, e.g., blood plasma and/or blood serum, interstitial liquid, cerebrospinal liquids, and similar.

Blood analysis is one of the most performed medical tests for providing an overview of a patient's health status. A blood sample can be drawn from a patient's body and stored in a container, e.g., a test tube, containing an anticoagulant to prevent clotting. For certain tests, an amount of a serum or plasma portion of the blood sample obtained from whole blood by fractionation (e.g., centrifugation) may be aspirated and used. A gel separator may be added to the blood sample inside the container to aid in the separation of a settled blood portion from the serum or plasma portion. After fractionation and after a subsequent de-capping process, the container may be transported to an appropriate analyzer that may extract, via aspiration, serum or plasma portion from the container and combine the serum or plasma portion with one or more reagents in a reaction vessel (e.g., cuvette). Analytical measurements may then be performed, e.g., using an interrogating radiation beam, or by using photometric and/or fluorometric absorption readings, or similar. Such analytical measurements allow for the determination of end-rate, point or other values, from which a concentration of constituent, analyte or other, may be determined.

Typically, the containers are labelled with one or more barcodes for handling by the LAS. The barcode label(s) may for example contain an accession number, test order, and/or other information, that may be correlated to demographic information that may be entered into a hospital's laboratory information system (LIS). For example, an operator may introduce the barcode-labeled containers into the LAS, such as in a rack, and the LAS may automatically transport the containers for pre-analytical stage processing such as centrifugation, decapping, and aliquot preparation, and/or similar. This pre-analytical stage processing may be carried out before the containers are clinically analysed or assayed by one or more analyzers, which may be part of the LAS or connected thereto.

A presence of certain interferents or artifacts in the biological sample, as a result of sample processing or patient disease condition, may signalize poor sample quality as it may potentially adversely affect the accuracy of the test results of the analyte or constituent measurement obtained from the analyzer. For example, a presence of hemolysis, icterus, and/or lipemia may affect the analyzer results of a blood sample. The sample quality, e.g., integrity of the serum or plasma portion of a blood sample, may be determined by an automated visual inspection method using image processing of an image captured by a camera from a viewpoint on the container. Specifically, the sample quality may be reflected in the color of the biological sample. The sample quality of the blood sample may be indicative of the presence of hemolysis, icterus and/or lipemia, each of which may be reflected in a different color or color spectrum of the biological sample.

The automated visual inspection method may be carried out prior to analysis at the one or more analyzers, i.e., at the pre-analytical stage processing, to determine the sample quality of the biological sample. However, in some instances, the one or more barcode labels adhered to the container may partially or fully occlude the view on the biological sample, so that the image of the container may not be optimal or not at all be usable for reliable visual inspection to accurately determine the sample quality of the biological sample. The LAS has no influence on how many and in which way the barcodes are labelled on the containers, which are being introduced to the LAS.

### SUMMARY OF THE INVENTION

The above problem is at least partially solved or alleviated by the subject matters of the independent claims of the present disclosure, wherein further examples are incorporated in the dependent claims.

According to an aspect of the present disclosure, there is provided a method for determining a sample quality of a biological sample inside an uncapped container, the method comprising:
- determining, by a data processing apparatus, at least one sample color of the biological sample based, e.g., the at least one sample color being determined based, on a color image showing at least part of a surface of the biological sample from a top view perspective on the container; and
- determining, by the data processing apparatus, the sample quality of the biological sample based on a comparison of the determined at least one sample color with at least one reference color.

Accordingly, the method of the first aspect of this disclosure solves the problem of obstructed views on the biological sample inside the container due to one or more barcode labels by capturing and processing a color image showing at least part of the surface of the biological sample from a top view perspective on the container. By processing the color image of this perspective for the determination of sample quality, the problem of obstructed view of the container, in particular in a side perspective view of the container, and hence the difficulty of determining the sample quality is solved. The determination of the sample quality is based on the color comparison with the color of the surface from the color image.

As described, both steps, the determining of the at least one sample color and the determining of the sample quality may be processed by a data processing system, which may be part of a lab(oratory) automation system (LAS). Hence, the method may in particular be a fully or at least partially computer-implemented method. This means that at least one, multiple or all of the steps of the method may be carried out by the data processing system. The data processing system may comprise one or more computers or computing units. The data processing system may be a data processing apparatus or device, e.g., in case of only one computer or multiple computers within the same apparatus or device. Alternatively, the data processing system may be a distributed computer system with several computers being in different locations and interconnected with one another, e.g., via a wireless network and/or the Internet. Different steps of the method may be carried out by the same or by different computers. A computer is herein understood as a data processing unit, which can carry out some, multiple or all steps as defined by the method.

The method may further comprise, in particular when only partially computer implemented, a step of obtaining, by a camera apparatus, the color image. This color image may then be received by the data processing system, e.g., by having the camera apparatus forward it to the data processing system. Accordingly, the data processing system and the camera apparatus may be connected with one another, e.g., wirelessly and/or via a wired connection. The camera apparatus may be an RGB camera.

Moreover, the method may comprise determining the at least one sample color, which may be multiple sample colors, in particular a sample color spectrum, based on the color image, in particular by the data processing system. For example, based on the color image, all different sample colors or one or more predominantly present sample colors, e.g., visible on at least a predetermined minimum area of the surface of the biological sample, or mean or arithmetic averages of all or one or more sample colors visible on the surface, may be determined as the at least one sample color. Also, the entire or part of the image may be obtained. Also, or alternatively, the entire or part of the surface with all sample colors or all sample colors may be determined and processed for the determination of the sample quality. The sample color may be, for example, determined and/or processed for the determination of the sample quality as part of the color image, as full image and/or any other representation of a color, e.g., a color code, such as an RGB color code. Similarly, the method may comprise determining the at least one reference color.

For example, the biological sample may be a body fluid sample. Further, for example, the biological sample may be a blood sample. However, the application of the method is not limited towards body fluid samples or blood samples and the biological sample may be of any other type, e.g., microorganisms, urine, interstitial liquid, cerebrospinal liquids, but not limited to these examples.

For example, the container may be a tube, which may have a greater tube elongation than tube diameter. In particular, the tube may be a collection tube for collecting the biological sample from a body, e.g., of a human or an animal. For example, the tube may be a blood collection tube. Generally, the container, in particular tube, may normally have a cap but may be uncapped, i.e., the cap may normally close the tube, but the cap may have been removed or omitted to not close the container to execute the method. A removing of the cap from the container may optionally be part of the method. In other words, the container may be open, in particular at the side, in particular top side, where the cap would normally be provided to close the container.

For example, the biological sample may comprise different materials separated from one another as material layers inside the container. For example, the different material layers in the biological sample may be obtained by fractionation, e.g., by centrifugation. The different materials may be in particular at least from a plasma, serum, red blood cell material and a separator or separator material, e.g., separation gel, for separating the material layers from one another, in particular in the case of a blood sample. Other material layers, e.g., a buffy coat, a whitish layer that may lay between the plasma or serum and the red blood cells, may also be present. Generally, the composition and appearance of the material layers may vary depending on the specific type of blood collection tube used and the processing techniques employed. The material layers may for example have different colors and thereby influence the sample color seen from above the container, in particular in the top view perspective on the container as seen in the color image. This may be taken into account for the determination of the sample quality. This may be in particular relevant when an upper material layer, e.g., plasma or serum, is relevant for the sample quality as opposed to a color of a non-centrifuged biological sample, e.g., a full blood sample. In the case of the upper material layer being relevant, it may be advantageous to calibrate the at least one sample color or the at least one reference color based on the color of the materials below that upper material layer.

The sample color seen on the surface of the biological sample is not necessarily the one of the biological sample and, similarly, the sample color of an upper material layer in case of a separated biological sample is not necessarily the one of the upper material layer. For example, the color of the surface may be influenced by any material or material layer below it, which may include material layers below an upper material layer of the biological sample as well as the container bottom wall and anything below it. Hence, the sample color of the surface of the biological sample may in effect be influenced by the materials below it and not only by its quality. In the method, the at least one sample color and the at least one reference color may be determined in a way such the accuracy of the determination of the sample quality is enhanced. For the determination, to exclude this effect, it is possible to calibrate the at least one sample color or the at least one reference color for this effect. Thereby, the method may use the true or actual sample color of the biological sample or the sample quality relevant material thereof for the comparison. Alternatively, the method may use at least one calibrated reference color, which may be calibrated for comparison to the at least one sample color seen on the surface by taking into account the effects of any material below the biological sample or the sample quality relevant material thereof.

For example, the at least one sample color or the at least one reference color may be determined based on a thickness of each one of the material layers and a color of each one of the different materials. For example, the at least one reference color may be determined, in particular calibrated, based on the thickness of each one of the material layers and the material color of each one of the different materials, before comparing it to the at least one sample color. That is, the reference color may be dependent on the composition of the biological sample, in particular its different material layers, and determining the correct reference color is important for accurately determining the sample quality, as explained above. The thickness of each one of the material layers may be known from a liquid level detection unit of the LAS, which may provide information about a fractionation or spun state and/or the thickness of the different material layers. The color may be known from the same or generally due to the type of material layer, e.g., being any of the aforementioned examples of materials, for example. Alternatively, the at least one sample color as such may be determined, in particular calibrated, in particular for a top or upper material (layer) in the biological sample, which may be the one seen from the top view perspective of the container in the color image and/or be the one, for which the sample quality is to be determined or which at least influences the sample quality of the entire biological sample. However, the color of this top or upper material layer may be dependent on the color of one or more material layers below it such that it may be advantageous to calibrate for this for an accurate determination of the sample quality. By knowing the material layer(s), in particular with their thickness(es) and material color(s), below the top or upper material layer, e.g., from the liquid level detection unit, the at least one sample color may be determined, e.g., by subtracting the influence of the material color(s) of the material layer(s) below the top or upper material layer on the sample color seen in the color image from the color as seen in the color image.

In an example, the thickness of each one of the material layers of the different materials may be determined based on a measurement, in particular laser measurement, of the different materials inside the container. For example, in the exemplary case of a laser measurement, one, two or more laser units may be used to carry out the laser measurement. The laser unit(s) may emit laser beams with different wavelengths on the container, the different materials may absorb the laser beams differently, and the different absorption characteristics of the different materials may be measured by measuring the laser beam going through the container. The laser unit(s) may be part of a liquid level detection unit as previously described. In particular the laser beam(s) may be emitted along the entire height of the biological sample inside the container. For example, the different materials may have different densities, which may have an influence on the absorption characteristics. The absorption characteristics or densities of the different material layers may be known and thus, based on the laser measurement, the thickness or height of each one of the different materials may be determined because the transition from one of the material layers to the other one will be recognized in terms of the different absorption characteristic.

In an example, the at least one sample color or the at least one reference color may be determined based on at least one of a thickness of a bottom wall of the container, a shape of the bottom wall, a color of the material below the container and a material color of the bottom wall. In other words, the thickness of the bottom wall, its shape, its color and/or a material color below the container may be considered to calibrate for the at least one sample color or the at least one reference color, in addition or alternatively to the thickness of each one of the material layers and a color of each one of the different materials, to ensure accurate determination of the sample quality.

For the determination of the sample quality of the biological sample, a comparison is made between the determined at least one sample color and the at least one reference color, which may optionally be obtained by the data processing system, e.g., from a data storage thereof. The comparison or the overall determination may be made in a deterministic way and/or be supported or made by artificial intelligence, e.g., a machine learning algorithm, which may provide the at least one reference color for comparison and/or have learned the at least one reference color and based on that determine the sample quality. The at least one reference color or several reference colors may be indicative of the sample quality or several different sample qualities, such that by comparing these to one another, e.g., by substantially matching these to one another, the data processing system may determine the sample quality. The sample quality may have any form or type such as but not limited to: a number, e.g., in the form of a grade, in the form of a classification, e.g., as pass or fail, as bad or good, and/or an instruction how to handle the biological sample (in particular as described further below herein in the form of assigning the container for analysis or flagging, inspecting and/or requesting resample of the biological sample), or similar. Further, the sample quality may be associated with certain types of defects for the particular type of biological sample.

For example, the sample quality may be determined to be indicative of at least one of a homolytic, icteric, lipemic or normal material inside the biological sample. Accordingly, the sample qualities to be determined may be associated with the different qualities or certain types of homolytic, icteric, lipemic or normal material inside the biological sample. This may be the case in particular for a blood sample. The normal material quality of the biological sample may be neither of homolytic, icteric or lipemic material. Each one of the homolytic, icteric, lipemic or normal material may be determined based on their color as seen from the top view perspective on the surface of the biological sample. The different materials typically have different colors that may be differentiated from one another and varying from the normal quality of a biological sample.

In an example, the method may comprise, based on the determined sample quality:
- assigning the container for an at least partially automated clinical chemistry or immunoassay analysis of the biological sample;
- assigning the container for an at least partially manual clinical chemistry or immunoassay analysis of the biological sample; or
- flagging, inspecting and/or requesting resampling of the biological sample.
For example, in case the sample quality is determined to be indicative of a normal material, the container may be assigned for an at least partially or fully automated clinical chemistry or immunoassay analysis of the therein contained biological sample. However, if the sample quality is determined to be indicative of at least one of a homolytic, icteric or lipemic material inside the biological sample, it may be assigned for an at least partially or fully manual clinical chemistry or immunoassay analysis of the biological sample contained therein or, alternatively, for flagging, inspecting and/or requesting resampling of the therein contained biological sample. Based on human analysis, the biological sample may be potentially processed in a way such that despite the poor sample quality, it may deliver useful results. For example, the flagging may be an operation by which the container is flagged for a further action, e.g., inspection. The inspection may be then done by a human, who may ultimately decide, whether the biological sample shall or can be analyzed or not. Further, resampling of the biological sample may be requested, if the sample quality is not sufficient for clinical chemistry or immunoassay analysis.

In an example, the at least one sample color may be a color spectrum comprising several sample colors and/or the at least one reference color may be a reference color spectrum comprising several reference colors. In other words, a spectrum of colors on the surface of the biological sample and similarly a spectrum of reference colors may be used for the determination of the sample quality, which makes the determination of the sample quality more robust and accurate.

In an example, a label may be adhered to the container. The label may be comprising identification information for the therein contained biological sample. The identification information may be in any form, e.g., as a machine-readable code such as but not limited to a bar code, text, and/or QR code, for example. The identification information may be in the form of patient information, doctor information, hospital information and/or similar. The label may, in particular at least partially or fully, cover the biological sample from a side view perspective on the container. Thereby, a camera apparatus positioned with a sideview perspective on the container may not be able to reliably or accurately determine the sample quality of the biological sample.

In an example, the color image may show a side view perspective on the container and the top view perspective on the container, in particular together in one single color image. The at least part of the surface of the biological sample shown in the color image may be from a reflection of a mirror. The mirror may be positioned above the space for having the container positioned therein and oriented towards a position of the camera apparatus and the space such that the camera apparatus is configured to obtain the color image to show a side view perspective on the container and a reflection of the mirror showing the at least part of the surface of the biological sample. Thereby, both, the side view perspective and the top view perspective may be captured in one single image and by one single camera apparatus, without requiring multiple camera apparatuses in an LAS. However, generally, the color image may be taken in any possible way such that it shows the top view perspective on or of the container, e.g., by means of a camera positioned above the container and arranged to take the image from the top perspective view, or, as mentioned, by taking an image of a mirror.

According to a second aspect of this disclosure, there is provided a computer program product comprising instructions which, when the program is executed by a computer, cause the computer to carry out the method according to the first aspect of this disclosure.

The computer program product may be a computer program as such, meaning a computer program consisting of or comprising a program code to be executed by the computer. Alternatively, the computer program product may be a product such as a data storage, in particular a computer-readable data storage medium, on which the computer program may be temporarily or permanently stored.

According to a third aspect of this disclosure, there is provided a data processing system configured to carry out the method according to the first aspect of this disclosure.

The data processing system may comprise one or more computers as previously described and, optionally, the computer program product of the second aspect of this disclosure.

According to a fourth aspect of this disclosure, there is provided a system for determining a sample quality of a biological sample inside a container, the system comprising:
- a space for having the container positioned therein;
- a camera apparatus, configured to obtain a color image showing at least part of a surface of the biological sample from a top view perspective on the container; and
- the data processing system of the third aspect of this disclosure for determining the sample quality of the biological sample.

For example, the system may be configured as an LAS, in particular at least for pre-analytical stage processing of the biological sample. The system may also be configured for analytical and/or post-analytical stage processing of the biological sample, e.g., further comprise an analyzer apparatus for clinical chemistry or immunoassay analysis of the biological sample based on the determined sample quality. Moreover, as previously described, the system may comprise a laser apparatus, which may have one or more laser units. The laser apparatus may form or be part of a liquid level detection unit. The camera apparatus on the other hand may form or be part of a tube inspection unit, which may have further tasks then herein described, e.g., to determine the type of container, whether the container is capped or not, and similar. Moreover, the system may comprise a gripper configured to position the container inside the space, e.g., picking it from a rack or tray of the system, which may be holding several containers. The mirror as previously mentioned may be attached to the gripper, for example. Further, the data processing system may be configured to inspect the container based on the color image. For example, the container may be inspected for potential damages, for its label, e.g., the identification information thereof, and others.

It is noted that the above aspects, examples and features may be combined with each other irrespective of the aspect involved.

The above and other aspects of the present disclosure will become apparent from and elucidated with reference to the examples described hereinafter.

### BRIEF DESCRIPTION OF THE DRAWINGS

Exemplary embodiments will be further described with reference to Figures, wherein:
Figure 1 shows a schematic illustration of a system for determining a sample quality of a biological sample inside a container.
Figure 2 shows a schematic illustration of an image of the container.
Figures 3a to 3d show schematic illustrations of different biological samples inside the container.
Figure 4 show a schematic illustration of a method for determining the sample quality of the biological sample inside the container.

The Figures are schematic only and not true to scale. In principle, identical or like parts, elements and/or steps are provided with identical or like reference numerals in the Figures.

### DETAILED DESCRIPTION OF THE INVENTION

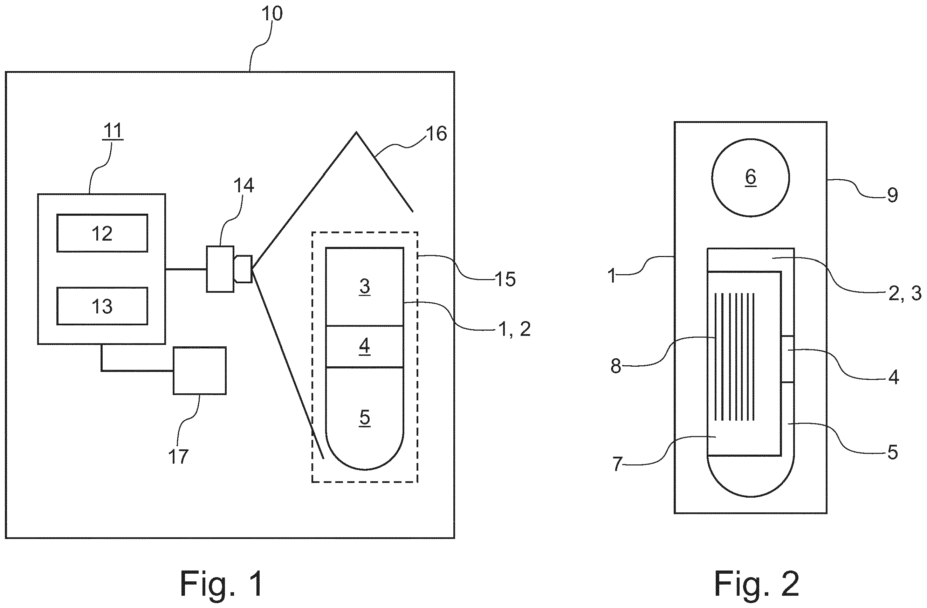

Figure 1 shows a system 10 for determining a sample quality of a biological sample 2 inside a container 1. The system 10 may be part of or configured as a laboratory automation system (LAS), which may be configured with equipment for pre-analytical, analytical and/or post-analytical processing of the biological sample 2. The biological sample 2 may in principle be any type of biological sample 2, including body fluid samples. In the example of this detailed description, it is exemplary assumed that the biological sample 2 is a blood sample 2.

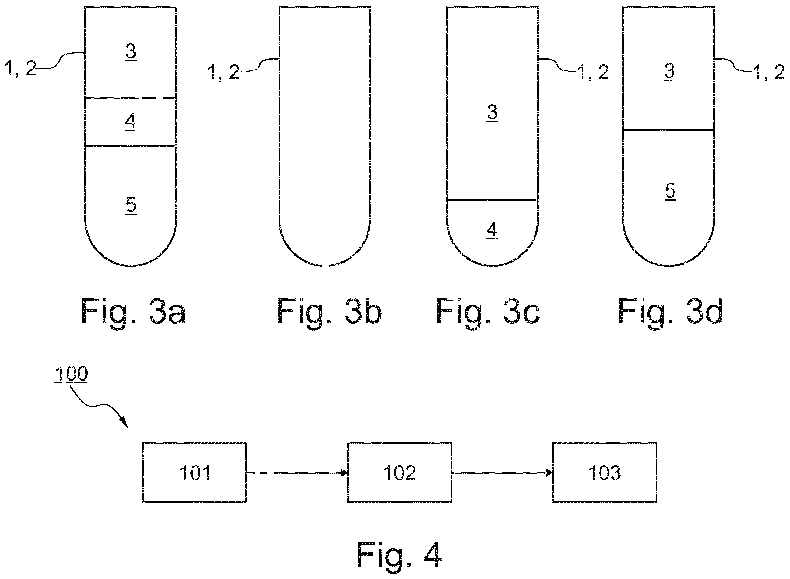

The system 10 comprises a space 15, in which one or more containers 1 may be positioned for carrying out the method 100 as shown exemplary in Fig. 4. A container 1, as shown in Fig. 1, may be positioned, or placed inside the space 15 by means of a gripper (not shown) or any other means, e.g., by gripping the container 1 from a rack or similar (not shown) and positioning it inside the space 15.

The system 10 further comprises a camera apparatus 14, which may be arranged such that it can obtain or capture a color image 9 of the container 1, in particular a color image 9 as exemplary shown in Fig. 2. The camera apparatus 14 may for example be configured as an RGB camera. The camera apparatus 14 may be part of a tube inspection unit for inspecting the container 1, which is herein exemplary embodied as a tube, in particular as a blood collection tube.

Further, the system 10 may comprise a data processing system 11, which may comprise a computer program product 12 and a computer 13. The camera apparatus 14 may be connected, wirelessly or via a wired connection, for example, to the data processing system 11 and submit the color image 9 to the data processing system 11. The data processing system 11 may process the color image 9 when carrying out the method 100 as shown in Fig. 4 by executing the computer program product 12 or the computer program stored on the computer program product 12.

The camera apparatus 14 is configured such that the obtained color image 9 shows at least part or all of a surface 6 of the biological sample 2 from a top view perspective on the container 1 as seen in the exemplary color image 9 shown in Fig. 2. The container 1 is uncapped. In the exemplary arrangement of the camera apparatus 14, a mirror 16 is being used. Here, the camera apparatus 14 is positioned to take the color image 9 from the side view perspective on the container 1, as also seen in the color image 9. Additionally, the mirror 16 positioned above the container 1 allows to capture, in the color image 9, the at least part or all of the surface 6 of the biological sample 2. Alternatively, there may be only one camera apparatus 14 positioned at the top of the container 1 and oriented to capture the color image 9 from the top view perspective of the container 1, or there may be two or more camera apparatuses 14 with different perspectives, e.g., the top view perspective and the side view perspective on the container 1.

As shown in Fig. 2, the container 1 may have a label 7 adhered thereto, in particular at a side thereof. The label 7 may for example comprise identification information 8 for the biological sample 2 inside the container 1, e.g., associated with patient data. The identification information 8 is shown to be a barcode in Fig. 2 as an example but text or other information may be alternatively or additionally provided on the label 7. The label 7 may, depending on its size, orientation or a number of labels 7 adhered to the container 1 at least partially or fully obstruct the side perspective view on the container 1, in particular by the camera apparatus 14. Hence, it may be difficult to determine the sample quality of the biological sample 2 inside the container 1 based on the side perspective view, wherein the sample quality may be determined based on the sample color, in particular sample color spectrum of the biological sample 2.

The top view perspective on the surface 6 of the biological sample 2, however, may not be obstructed by the one or more labels 7, and thus be reliably used for determining the sample quality, in particular in a case where the container 1 is not capped or has been uncapped during processing by the system 1.

For the purpose of sample quality determination of the example of the blood sample 2, it may be beneficial to consider the configuration of the blood sample 2, in particular in terms of a possible separation of its material or, in other words, the therein contained materials 3, 4, 5, which may have been separated from one another, e.g., by centrifugation and/or using a separator, e.g., a separation gel as shown as the material 4 in the examples of Figs. 3a and 3c Different examples of such configurations of blood samples 2 in containers 1 are shown in Figs. 3a to 3d.

In the exemplary blood samples 2 of Figs. 1, 2 and 3a, the blood sample 2 has been centrifuged using a separator as material 4, forming an intermediate material layer in the blood sample 2. The top or upper material 3 may be or comprise for example serum or plasma. The lower or bottom material 5 may be or comprise for example red blood cells. Here, the top or upper material 3 may be of interest for determining the sample quality of the blood sample 2 as it may be homolytic, icteric, lipemic or normal, which may be determined based on its color spectrum.

Similarly, the sample quality of the blood sample 2 may be determined for other configurations as shown in Figs. 3b, 3c and 3d. In Fig. 3b, the blood sample 2 is a full blood sample. In Fig. 3c, the blood sample 2 is not centrifuged with the separator being at the bottom as material 4. In Fig. 3d, the blood sample 2 is centrifuged without use of a separator.

The configuration of the blood sample 2 may be determined by a laser apparatus 17 of the system 10, for example, which may have one or more laser units and is only shown herein schematically. The absorption characteristic of the different materials 3, 4, 5 on the laser beam or laser beams emitted by the laser apparatus 17 may be measured (not shown) and therefrom, it may be determined which configuration the blood sample 2 has. The laser apparatus 17 may be part of a liquid level detection unit of the system 10, for example.

For determining the sample quality by the method 100, in a first step 101, a sample color spectrum of the blood sample 2 based on the color image 9 from Fig. 2 is determined by the data processing system 11, for which purpose the camera apparatus 14 may obtain the color image 9 by capturing it. In a second step 102, the sample quality of the blood sample 2 may be determined by the data processing system 11 based on a comparison of the determined sample color spectrum with one or more reference color spectrums, each one of which may be indicative of different sample quality, in particular of a homolytic, icteric, lipemic or normal blood sample 2.

It may be desirable to determine the sample color spectrum or the one or more reference color spectrums based on the configuration of the blood sample 2, in particular its separation into different materials 3, 4, 5, as this generally has an influence on the color spectrum seen in the color image 9 and the color of interest in the case of a centrifuged blood sample 2 may be only the one of one material layer, e.g., of the upper material 3.

For example, Fig. 3b shows a full blood sample 2 inside the container 1. Here, the color spectrum as seen in the color image 9 may be compared to the one or more reference color spectrums to determine the sample quality. Optionally, the sample color spectrum or the reference color spectrum may be determined, in particular calibrated, based on at least one of a thickness of a bottom wall of the container 1, a shape of the bottom wall, a color of the material below the container 1 and a material color of the bottom wall, which may be for example determined by the camera apparatus 14 and/or the laser apparatus 17. For example, the sample color spectrum may be determined such that the effect of the colors below the full blood sample 2, i.e., of the material below the container 1 and of the material of the container 1, which may be influenced by the thickness and shape of the container 1, is taken into consideration, e.g., by deducting their effect on the color of the full blood sample 2. Alternatively, the effect of the colors below the full blood sample 2 may be taken into consideration for the one or more reference color spectrums, e.g., by adding their effect on the reference color spectrums for the particular container 1. Thereby, it may be ensured that only the color of the full blood sample 2 or one or more references thereof are taken into account in the determination of the sample quality and the container 1 or material below the container 1 does not negatively affect the accuracy of determination of the sample quality.

Figs. 3a, 3c and 3d on the other hand show different material layers in the blood sample 2, where only the upper material 3, which may be the serum or plasma, may be of interest to determine the sample quality, e.g., the upper material 3 may be homolytic, icteric, lipemic or normal and it shall be determined which of these is the case for the particular blood sample 2. Here, besides the color, thickness and shape of the bottom wall of the container 1 as well as the color of the material below the container 1, the layers of the bottom or intermediate material 4, 5 below the upper material 3 may influence the color spectrum of the surface 6 of the blood sample 2. Hence, the sample color spectrum or the one or more reference color spectrums may be determined based on a thickness of each one of the material layers and a color of each one of the different materials 3, 4, 5. For example, the sample color spectrum may be determined such that the effect of the colors of the material 4, 5 below the upper material 3 is taken into consideration, e.g., by deducting their effect on the color of the upper material 3. Alternatively, the effect of the colors below the upper material 3 may be taken into consideration for the one or more reference color spectrums, e.g., by adding their effect on the reference color spectrums for the particular container 1. Thereby, it may be ensured that only the color of the quality relevant upper material 3 or one or more references thereof is taken into account in the determination of the sample quality and the color of the bottom or intermediate material 4, 5 does not negatively affect the accuracy of determination of the sample quality.

In a consequent step 103, depending on the determined sample quality, the method 100 may comprise assigning the container 1 for an at least partially automated clinical chemistry or immunoassay analysis of the biological sample 2, assigning the container 1 for an at least partially manual clinical chemistry or immunoassay analysis of the biological sample 2, or flagging, inspecting and/or requesting resampling of the biological sample 2.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practicing the claimed invention, from a study of the drawings, the disclosure, and the claims.

As used herein, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Further, as used herein, the phrase "at least one" or similar, e.g., "one or more of', in reference to a list of one or more entities should be understood to mean at least one entity selected from any one or more of the entities in the list of entities, but not necessarily including at least one of each and every entity specifically listed within the list of entities and not excluding any combinations of entities in the list of entities. This definition also allows that such entities may optionally be present other than the entities specifically identified within the list of entities to which the phrase "at least one" or similar refers, whether related or unrelated to those entities specifically identified. Thus, as a non-limiting example, "at least one of A and B" (or, equivalently, "at least one of A or B" or, equivalently "at least one of A and/or B" or, equivalently "one or more of A and B", "one or more of A or B", or "one or more of A and/or B") may refer, in one example, to at least one, optionally including more than one, A, with no B present (and optionally including entities other than B); in another example, to at least one, optionally including more than one, B, with no A present (and optionally including entities other than A); in yet another example, to at least one, optionally including more than one, A, and at least one, optionally including more than one, B (and optionally including other entities). In other words, the phrases "at least one," "one or more," and "and/or" are open-ended expressions that are both conjunctive and disjunctive in operation. For example, each of the expressions "at least one of A, B, and C," "at least one of A, B, or C," "one or more of A, B, and C," "one or more of A, B, or C," and "A, B, and/or C" may mean A alone, B alone, C alone, A and B together, A and C together, B and C together, A, B, and C together, and optionally any of the above in combination with at least one other entity.

As used herein, the phrase "being indicative of' may for example mean "reflecting" and/or "comprising". Accordingly, an entity, element and/or step referred to herein as "being indicative of [...]" can be synonymously or interchangeably used herein with one, two or all of said entity, element and/or step "comprising [...]" and said entity, element and/or step "reflecting [...]".

Further, as used herein, phrases such as "based on", "related" or "relating", "associated" and similar are not to be seen exclusively in terms of the entities, elements and/or steps to which they are referring, unless otherwise stated. Instead, these phrases are to be understood inclusively, unless otherwise stated, in that, for example, an entity, element or step referring by any of these phrases or similar, e.g., being "based on", an or another entity, element or step, does not exclude that the respective entity, element or step may be further or also "based on" any other entity, element or step than the one to which it refers.

Any designation of methods and steps as first, second, etc. as provided herein is merely intended to make the methods and their steps referenceable and distinguishable from one another. By no means does the designation of methods and steps constitute a limitation of the scope of this disclosure. For example, when this disclosure describes a third step of a method, a first or second step of the method do not need to be present yet alone be performed before the third step unless they are explicitly referred to as being required per se or before the third step. Moreover, the presentation of methods or steps in a certain order is merely intended to facilitate one example of this disclosure and by no means constitutes a limitation of the scope of this disclosure. Generally, unless no explicitly required order is being mentioned, the methods and steps may be carried out in any feasible order. Specifically, the terms first, second, third or (a), (b), (c) and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In the context of the present invention any numerical value indicated is typically associated with an interval of accuracy that the person skilled in the art will understand to still ensure the technical effect of the feature in question. As used herein, the deviation from the indicated numerical value is in the range of ± 10%, and preferably of ± 5%. The aforementioned deviation from the indicated numerical interval of ± 10%, and preferably of ± 5% is also indicated by the terms "about" and "approximately" used herein with respect to a numerical value.

Any reference signs in the claims should not be construed as limiting the scope.

## Claims

1. A method (100) for determining a sample quality of a biological sample (2) inside an uncapped container (1), the method (100) comprising:
- determining, by a data processing system (11), at least one sample color of the biological sample (2) based on a color image (9) showing at least part of a surface (6) of the biological sample (2) from a top view perspective on the container (1); and
- determining, by the data processing system (11), the sample quality of the biological sample (2) based on a comparison of the determined at least one sample color with at least one reference color.

2. The method (100) of claim 1, wherein the biological sample (2) is a body fluid sample.

3. The method (100) of claim 1 or 2, wherein the biological sample (2) is a blood sample.

4. The method (100) of any one of the previous claims, wherein the biological sample (2) comprises different materials (3, 4, 5) separated from one another as material layers inside the container (1), wherein the different materials (3, 4, 5) are in particular at least from a plasma, serum, red blood cell material and a separator for separating the material layers from one another.

5. The method (100) of claim 4, wherein the at least one sample color or the at least one reference color is determined based on a thickness of each one of the material layers and a color of each one of the different materials (3, 4, 5).

6. The method (100) of claim 5, wherein the thickness of each one of the material layers of the different materials (3, 4, 5) is determined based on a measurement, in particular laser measurement, of the different materials (3, 4, 5) inside the container (1).

7. The method (100) of any one of the previous claims, wherein the at least one sample color or the at least one reference color is determined based on at least one of a thickness of a bottom wall of the container (1), a shape of the bottom wall, a color of the material below the container (1) and a material color of the bottom wall.

8. The method (100) of any one of the previous claims, wherein the sample quality is determined to be indicative of at least one of a homolytic, icteric, lipemic or normal material inside the biological sample (2).

9. The method (100) of any one of the previous claims, wherein the method (100) comprises, based on the determined sample quality:
- assigning the container (1) for an at least partially automated clinical chemistry or immunoassay analysis of the biological sample (2);
- assigning the container (1) for an at least partially manual clinical chemistry or immunoassay analysis of the biological sample (2); or
- flagging, inspecting and/or requesting resampling of the biological sample (2).

10. The method (100) of any one of the previous claims, wherein the at least one sample color is a color spectrum comprising several sample colors and/or the at least one reference color is a reference color spectrum comprising several reference colors.

11. The method (100) of any one of the previous claims, wherein a label (7) is adhered to the container (1), the label (7) comprising identification information (8) for the therein contained biological sample (2), wherein the label (7) covers the biological sample (2) from a side view perspective on the container (1).

12. The method (100) of any one of the previous claims, wherein the color image (9) shows a side view perspective on the container (1) and the top view perspective on the container (1), wherein the at least part of the surface (6) of the biological sample (2) shown in the color image (9) is from a reflection of a mirror (16).

13. A computer program product (12) comprising instructions which, when the program is executed by a computer (13), cause the computer (13) to carry out the method (100) of any one of the previous claims.

14. A data processing system (11) comprising means for carrying out the method (100) of any one of the claims 1 to 12.

15. A system (10) for determining a sample quality of a biological sample (2) inside a container (1), the system (10) comprising:
- a space (15) for having the container (1) positioned therein;
- a camera apparatus (14), configured to obtain a color image (9) showing at least part of a surface (6) of the biological sample (2) from a top view perspective on the container (1); and
- the data processing system (11) of claim 14 for determining the sample quality of the biological sample (2).
